# EUROPEAN PATENT SPECIFICATION

(11) **EP 2 992 006 B1**
(45) Date of publication and mention of the grant of the patent: **28.03.2018**
(21) Application number: 13883373.6
(22) Date of filing: 28.04.2013
(51) Int. Cl.: C07K 14/415, C12N 9/10, C12N 15/54, C12N 15/63, C12N 5/10, A01H 5/00, C12N 15/82

(54) **NOVEL PROTEIN AND GENE RELATED TO FLAVONOID O-METHYLTRANSFERASE (FOMT) AND THEIR USES THEREFORE**
NEUARTIGES PROTEIN UND GEN IN ZUSAMMENHANG MIT FLAVONOID-O-METHYLTRANSFERASE (FOMT) UND DEREN VERWENDUNGEN DAFÜR
NOUVELLE PROTÉINE ET GÈNE ASSOCIÉ À UNE FLAVONOÏDE O-MÉTHYLTRANSFÉRASE (FOMT) ET LEURS UTILISATIONS ASSOCIÉES

(43) Date of publication of application: 09.03.2016
(73) Proprietor: Institute of Botany of The Chinese Academy of Sciences, Beijing 100093 (CN)
(72) Inventor: WANG, Liangsheng, Beijing 100093 (CN); DU, Hui, Beijing 100093 (CN); SHU, Qingyan, Beijing 100093 (CN)
(74) Representative: Grünecker Patent- und Rechtsanwälte PartG mbB
(86) International application number: PCT/CN2013/074973
(87) International publication number: WO 2014/176732

(56) References cited:
- EP-A2- 2 096 172
- ALEXANDRE FOURNIER-LEVEL ET AL: "Genetic mechanisms underlying the methylation level of anthocyanins in grape (Vitis vinifera L.)", BMC PLANT BIOLOGY, BIOMED CENTRAL, LONDON, GB, vol. 11, no. 1, 15 December 2011 (2011-12-15), page 179, XP021093321, ISSN: 1471-2229, DOI: 10.1186/1471-2229-11-179
- DAQIU ZHAO ET AL: "Flower color diversity revealed by differential expression of flavonoid biosynthetic genes and flavonoid accumulation in herbaceous peony (Pall.)", MOLECULAR BIOLOGY REPORTS ; AN INTERNATIONAL JOURNAL ON MOLECULAR AND CELLULAR BIOLOGY, KLUWER ACADEMIC PUBLISHERS, DO, vol. 39, no. 12, 10 October 2012 (2012-10-10), pages 11263-11275, XP035132926, ISSN: 1573-4978, DOI: 10.1007/S11033-012-2036-7
- MA, CHENGYING ET AL.: 'Cloning and prokaryotic expression of flavonoid O-methyltransferase from Camellia sinensis' SCIENTIA AGRICULTURA SINICA vol. 46, no. 2, 16 January 2013, pages 325 - 333, XP008180838
- ZHANG, JINGJING ET AL. RECENT ADVANCES IN FLOWER COLOR RESEARCH OF TREE PEONY vol. 33, no. 6, 30 December 2006, pages 1383 - 1388, XP055280302
- SHU, QING Y. ET AL.: 'Functional annotation of expressed sequence tags as a tool to understand the molecular mechanism controlling flower bud development in tree peony' PHYSIOLOGIA PLANTARUM vol. 135, 04 February 2009, pages 436 - 449, XP055280182

### SEQUENCE LISTING

<110> INSTITUTE OF BOTANY, THE CHINESE ACADEMY OF SCIENCES
   <120> Novel protein and gene related to flavonoid O-methyltransferase (FOMT) and their uses therefore
   <130> PC130103CN/ZWS
   <160> 22
   <170> PatentIn version 3.5
<210> 1
   <211> 708
   <212> DNA
   <213> *Paeonia suffruticosa* cv. ' Gunpohden'
   <400> 1
   atggccgagt cagataggaa gggtattctt aaaagtgaag cccttttgaa gtacatatta      60
   gaaactagtg tctaccccag ggaacatgaa caactcaagc aactcagaca ggctaccgtc      120
   gacaagtaca aatactggag tgtgatgaat gtgcctgctg atgaagggca gttcctctca      180
   atgcttttga agattatgaa tgcaaagaag actatcgagg ttggagtctt tactggttat      240
   tctcttctca ctgccgctct tgctctgcct accgatgcca aaataacagc aatcgatcca      300
   gacggggaag cctacgagac tggacttcca tttattaaga aggctggagt ggagcataag      360
   attaactaca ttcaagcaga tgcaatgtca gttttagatg atcttgtgac taatgggaag      420
   gaagaaggga gctttgattt tgcatttgtg gatgcggaca aggaaaacta tgccaaatat      480
   catgagccac tgatgaagtt ggttaaaatt ggtggaataa ttggctatga caatacattg      540
   tggtttggtt cagttgcact ctcggacggt gatgagatga aggaatttca ggaaaagggc      600
   aggcgcatct ttagagagtt caacactttc ttagctgctg atcctcgtat tgaattagct      660
   cacatttcca ttggggacgg tctcgctctc tgtaggcgcc tctattag                   708
<210> 2
   <211> 708
   <212> DNA
   <213> *Paeonia tenuifolia*
   <400> 2
   atggccgagt cagataggaa gggtattctt aaaagtggag cccttttgaa gtacatatta    60
   gaaactagtg tctaccccag ggaacatgaa caactcaagc aactcagaca ggctaccgtc   120
   gacaagtaca aatactggag tgtgatgaat gtgcctgctg atgaagggca gttcctctca   180
   atgcttttga agattatgaa tgcaaaaaag actatcgagg ttggagtctt tactggttat   240
   tctcttctca ctaccgctcg tgctctgcct accgatgcca aaataacagc aatcgatcca   300
   gacggagaag cctacgagac tggacttcca tttattaaga aggctggagt ggagcataag   360
   attaactaca ttcaagcaga tgcaatgtca gttttagatg atcttgtgac taatgggaag   420
   gaagaaggga gctttgattt tgcatttgtg gatgctgaca aggaaaacta tgccaaatat   480
   catgagccac tgatgaagct ggttaaaatt ggtggaataa ttggctatga caatacattg   540
   tggtttggtt cagttgcact ctcggacggt gatgagatga aggaatttca ggaaaagggc   600
   aggcgcatct ttacagagtt caacactttc ttagctgctg atcctcgtat tgaattagct   660
   cacatttcca ttggggacgg cctcgccctc tgtaggcgcc tctattag                708
<210> 3
   <211> 235
   <212> PRT
   <213> *Paeonia suffruticosa* cv. ' Gunpohden'
   <400> 3
   Met Ala Glu Ser Asp Arg Lys Gly Ile Leu Lys Ser Glu Ala Leu Leu
   1 5 10 15
   Lys Tyr Ile Leu Glu Thr Ser Val Tyr Pro Arg Glu His Glu Gln Leu
   20 25 30
   Lys Gln Leu Arg Gln Ala Thr Val Asp Lys Tyr Lys Tyr Trp Ser Val
   35 40 45
   Met Asn Val Pro Ala Asp Glu Gly Gln Phe Leu Ser Met Leu Leu Lys
   50 55 60
   Ile Met Asn Ala Lys Lys Thr Ile Glu Val Gly Val Phe Thr Gly Tyr
   65 70 75 80
   Ser Leu Leu Thr Ala Ala Leu Ala Leu Pro Thr Asp Ala Lys Ile Thr
   85 90 95
   Ala Ile Asp Pro Asp Gly Glu Ala Tyr Glu Thr Gly Leu Pro Phe Ile
   100 105 110
   Lys Lys Ala Gly Val Glu His Lys Ile Asn Tyr Ile Gln Ala Asp Ala
   115 120 125
   Met Ser Val Leu Asp Asp Leu Val Thr Asn Gly Lys Glu Glu Gly Ser
   130 135 140
   Phe Asp Phe Ala Phe Val Asp Ala Asp Lys Glu Asn Tyr Ala Lys Tyr
   145 150 155 160
   His Glu Pro Leu Met Lys Leu Val Lys Ile Gly Gly Ile Ile Gly Tyr
   165 170 175
   Asp Asn Thr Leu Trp Phe Gly Ser Val Ala Leu Ser Asp Gly Asp Glu
   180 185 190
   Met Lys Glu Phe Gln Glu Lys Gly Arg Arg Ile Phe Arg Glu Phe Asn
   195 200 205
   Thr Phe Leu Ala Ala Asp Pro Arg Ile Glu Leu Ala His Ile Ser Ile
   210 215 220
   Gly Asp Gly Leu Ala Leu Cys Arg Arg Leu Tyr
   225 230 235
<210> 4
   <211> 235
   <212> PRT
   <213> *Paeonia tenuifolia*
   <400> 4
   Met Ala Glu Ser Asp Arg Lys Gly Ile Leu Lys Ser Gly Ala Leu Leu
   1 5 10 15
   Lys Tyr Ile Leu Glu Thr Ser Val Tyr Pro Arg Glu His Glu Gln Leu
   20 25 30
   Lys Gln Leu Arg Gln Ala Thr Val Asp Lys Tyr Lys Tyr Trp Ser Val
   35 40 45
   Met Asn Val Pro Ala Asp Glu Gly Gln Phe Leu Ser Met Leu Leu Lys
   50 55 60
   Ile Met Asn Ala Lys Lys Thr Ile Glu Val Gly Val Phe Thr Gly Tyr
   65 70 75 80
   Ser Leu Leu Thr Thr Ala Arg Ala Leu Pro Thr Asp Ala Lys Ile Thr
   85 90 95
   Ala Ile Asp Pro Asp Gly Glu Ala Tyr Glu Thr Gly Leu Pro Phe Ile
   100 105 110
   Lys Lys Ala Gly Val Glu His Lys Ile Asn Tyr Ile Gln Ala Asp Ala
   115 120 125
   Met Ser Val Leu Asp Asp Leu Val Thr Asn Gly Lys Glu Glu Gly Ser
   130 135 140
   Phe Asp Phe Ala Phe Val Asp Ala Asp Lys Glu Asn Tyr Ala Lys Tyr
   145 150 155 160
   His Glu Pro Leu Met Lys Leu Val Lys Ile Gly Gly Ile Ile Gly Tyr
   165 170 175
   Asp Asn Thr Leu Trp Phe Gly Ser Val Ala Leu Ser Asp Gly Asp Glu
   180 185 190
   Met Lys Glu Phe Gln Glu Lys Gly Arg Arg Ile Phe Thr Glu Phe Asn
   195 200 205
   Thr Phe Leu Ala Ala Asp Pro Arg Ile Glu Leu Ala His Ile Ser Ile
   210 215 220
   Gly Asp Gly Leu Ala Leu Cys Arg Arg Leu Tyr
   225 230 235
<210> 5
   <211> 33
   <212> DNA
   <213> Artificial Sequence
   <220>
   <223> *BamH*I-forward
   <400> 5
   taggatccat ggccgagtca gataggaagg gta 33
<210> 6
   <211> 34
   <212> DNA
   <213> Artificial Sequence
   <220>
   <223> *Xho*I-reverse
<400> 6
   ttactcgagc atggctaata gaggcgccta caga 34
<210> 7
   <211> 38
   <212> DNA
   <213> Artificial Sequence
   <220>
   <223> *Nde*I-forward
   <400> 7
   gggaattcca tatgatggcc gagtcagata ggaagggt 38
<210> 8
   <211> 37
   <212> DNA
   <213> Artificial Sequence
   <220>
   <223> *BamH*I-reverse
   <400> 8
   ttacgcggat cccatggcta atagaggcgc ctacaga 37
<210> 9
   <211> 25
   <212> DNA
   <213> Artificial Sequence
   <220>
   <223> *PtFOMT* G13E-forward
   <400> 9
   tattcttaaa agtgaagccc ttttg 25
<210> 10
   <211> 25
   <212> DNA
   <213> Artificial Sequence
   <220>
   <223> *PtFOMT* G13E-reverse
   <400> 10
   tcacttttaa gaataccctt cctat 25
<210> 11
   <211> 25
   <212> DNA
   <213> Artificial Sequence
   <220>
   <223> *PtFOMT* T85A-forward
   <400> 11
   ttattctctt ctcactgccg ctcgt 25
<210> 12
   <211> 26
   <212> DNA
   <213> Artificial Sequence
   <220>
   <223> *PtFOMT* T85A-reverse
   <400> 12
   cagtgagaag agaataacca gtaaag 26
<210> 13
   <211> 25
   <212> DNA
   <213> Artificial Sequence
   <220>
   <223> *PtFOMT* R87L-forward
   <400> 13
   tcactaccgc tcttgctctg cctac 25
<210> 14
   <211> 25
   <212> DNA
   <213> Artificial Sequence
   <220>
   <223> *PtFOMT* R87L-reverse
   <400> 14
   agagcggtag tgagaagaga ataac 25
<210> 15
   <211> 25
   <212> DNA
   <213> Artificial Sequence
   <220>
   <223> *PtFOMT* T205R-forward
   <400> 15
   ggcgcatctt tagagagttc aacac 25
<210> 16
   <211> 26
   <212> DNA
   <213> Artificial Sequence
<220>
   <223> *PtFOMT* T205R-reverse
   <400> 16
   ctaaagatgc gcctgccctt ttcctg 26
<210> 17
   <211> 25
   <212> DNA
   <213> Artificial Sequence
   <220>
   <223> *PsFOMT* L87R-forward
   <400> 17
   cttctcactg ccgctcgtgc tctgc 25
<210> 18
   <211> 26
   <212> DNA
   <213> Artificial Sequence
   <220>
   <223> *PsFOMT* L87R -reverse
   <400> 18
   cgagcggcag tgagaagaga ataacc 26
<210> 19
   <211> 25
   <212> DNA
   <213> Artificial Sequence
   <220>
   <223> *PsFOMT* L87A-forward
   <400> 19
   tctcactgcc gctgctgctc tgcct 25
<210> 20
   <211> 26
   <212> DNA
   <213> Artificial Sequence
   <220>
   <223> *PsFOMT* L87A-reverse
   <400> 20
   gcagcggcag tgagaagaga ataacc 26
<210> 21
   <211> 24
   <212> DNA
   <213> Artificial Sequence
   <220>
   <223> PsFOMT-forward
   <400> 21
   atggccgagt cagataggaa gggt 24
<210> 22
   <211> 23
   <212> DNA
   <213> Artificial Sequence
   <220>
   <223> PsFOMT-reverse
   <400> 22
   taaaatgatc cttctgccat gct 23

## Description

### Field of the invention

The present invention relates to novel proteins and genes and their uses thereof. More specifically, the present invention relates to proteins involved in flavonoid *O*-methyltransferase (FOMT), and genes encoding these proteins, and their uses thereof.

### Background of the invention

Methyltransferase (EC 2.1) catalyzes an alkylation reaction transferring transfers an activated methyl group from *S*-adenosylmethionine (SAM or AdoMet) which is the most commonly methyl donor molecular, to *N*-, *C*-, *O*-*, S*-nucleophiles in DNA, RNA, protein, polysaccharide, lipid and a range of small molecules (Cantoni, G.L., Biological Methylation - Selected Aspects. Annual Review of Biochemistry, 1975. 44: p. 435-451.). Methyltranferases are classified by the type of nucleophiles, i.e. *O*-methyltransferase and *C*-methyltransferase. Additionally, they can also be sorted by substrates which are methylated. Methylation plays an important role in many essential biological processes. Methylation of DNA regulates gene expression, methylation of phospholipids keeps the membrane fluid, and the methylation of hormones, neurotransmitters and polyphenols etc. is crucial for the regulation of signal-transduction and self-defense (Fontecave, M., M. Atta, and E. Mulliez, S-adenosylmethionine: nothing goes to waste. Trends in Biochemical Sciences, 2004. 29(5): p. 243-249.). Despite the widespread function of methylation, the activity of methyltransferases generally works in a specific way regarding to its various function (Klimasauskas, S. and E. Weinhold, A new tool for biotechnology: AdoMet-dependent methyltransferases. Trends in Biotechnology, 2007. 25(3): p. 99-104.).

There are various SAM-*O*-methyltransferases (OMT) in plant, which differ in their selectivity with respect to the stereochemistry of the methyl acceptor molecules (e.g. phenylpropanoids, flavonoids and benzylisoquinoline alkaloids), and they can be classified into two types. Type I includes a group of low molecular weight (23-27 kD) and cation dependent OMTs. Most of them have been shown to be specific methylation for caffeoyl coenzyme A esters of phenylpropanoids (CCoAOMTs), and suggested to be key enzymes in the biosynthesis of monolignols, the precursors of gymnosperm and angiosperm lignins (Ye, Z.H., et al., An alternative methylation pathway in lignin biosynthesis in Zinnia. Plant Cell, 1994. 6(10): p. 1427-1439.). Type II consists of higher molecular weight (38-43 kD), cation independent homodimeric OMTs which methylate large family members including caffeic acid, flavones, isoflavone, coumarin and alkaloid OMTs (Frick, S. and T.M. Kutchan, Molecular cloning and functional expression of O-methyltransferases common to isoquinoline alkaloid and phenylpropanoid biosynthesis. Plant Journal, 1999. 17(4): p. 329-39.; Ibrahim, R.K., A. Bruneau, and B. Bantignies, Plant O-methyltransferases: molecular analysis, common signature and classification. Plant Molecular Biology, 1998. 36(1): p. 1-10.; Ibdah, M., et al., A novel Mg2+-dependent O-methyltransferase in the phenylpropanoid metabolism of Mesembryanthemum crystallinum. Journal of Biological Chemistry, 2003. 278(45): p. 43961-43972.; Hugueney, P., et al., A novel cation-dependent O-methyltransferase involved in anthocyanin methylation in grapevine. Plant Physiology, 2009. 150(4): p. 2057-2070.).

However, Ibdah et al. (2003) reported that a novel Mg²⁺-dependent PFOMT from *Mesembryanthemum crystallinum* (ice plant) (Ibdah, M., et al., A novel Mg2+-dependent O-methyltransferase in the phenylpropanoid metabolism of Mesembryanthemum crystallinum. Journal of Biological Chemistry, 2003. 278(45): p. 43961-43972.), with a molecular weight of 26.6 kD and high similarity to type I OMTs, was specific for flavonols and caffeoyl-CoA. Therefore, a novel subclass with diverse substrates not only restricted to lignin synthesis within type I OMTs was proposed. A patent of Florigen (2003) revealed a genetic sequence encoding a type I-like polypeptide with flavonoid methyltransferase (FMT) activity from *Petunia*, *Torenia*, or *Fuchsia* (Brugliera, F., et al., *Genetic sequences having methyltransferase activity and uses therefor.* 2003, WO Patent 2,003,062,428.), and anthocyanins (delphinidin, cyanidin derivates) can be used as the substrates, conducted by crude protein assays. Lee et al. (2008) cloned two genes *ROMT15* and *ROMT17* encoding small molecular OMTs with substrate specificity for tricetin, luteolin, myricetin, and caffeoyl-CoA (Lee, Y.J., et al., Cation dependent O-methyltransferases from rice. Planta, 2008. 227(3): p. 641-7.). Two genes (*VvAOMT* and *FAOMT*) from different grapevine cultivars were verified to encoding anthocyanin OMTs (Hugueney, P., et al., A novel cation-dependent O-methyltransferase involved in anthocyanin methylation in grapevine. Plant Physiology, 2009. 150(4): p. 2057-2070.; Lucker, J., S. Martens, and S.T. Lund, Characterization of a Vitis vinifera cv. Cabernet Sauvignon 3,5'-O-methyltransferase showing strong preference for anthocyanins and glycosylated flavonols. Phytochemistry, 2010. 71(13): p. 1474-1484.), their enzymatic activities were examined *in vitro* or *in vivo*, with preference to cyanidin 3-*O*-glucoside, delphinidin 3-*O*-glucoside, and quercetin 3-*O*-glucoside. The methylated positions were of 3'-OH or 3'- & 5'-*O*- B ring of anthocyanidin, indicating that the protein can act as A3'5'OMT. Kovinich (2011) isolated a gene of *OMT5* from black soybean by transcriptome analysis, which was validated to be an anthocyanin 3'-*O*-methyltransferase *in vitro* (Kovinich, N., et al., Combined analysis of transcriptome and metabolite data reveals extensive differences between black and brown nearly-isogenic soybean (Glycine max) seed coats enabling the identification of pigment isogenes. BMC Genomics, 2011. 12.). Akita et al. (2011) revealed a gene (*CkmOMT2*) from purple-flowered fragrant cyclamen, and the enzyme assay *in vitro* demonstrated that CkmOMT2 was responsible for the methylation of 3' or 3',5'-*O*- of anthocyanin substrates (Akita, Y., et al., Isolation and characterization of the fragrant cyclamen O-methyltransferase involved in flower coloration. Planta, 2011. 234(6): p. 1127-1136.). Therefore, the subclass in type I OMTs has been validated by above researches to be cation-dependent small molecular OMT with preference for flavonoids.

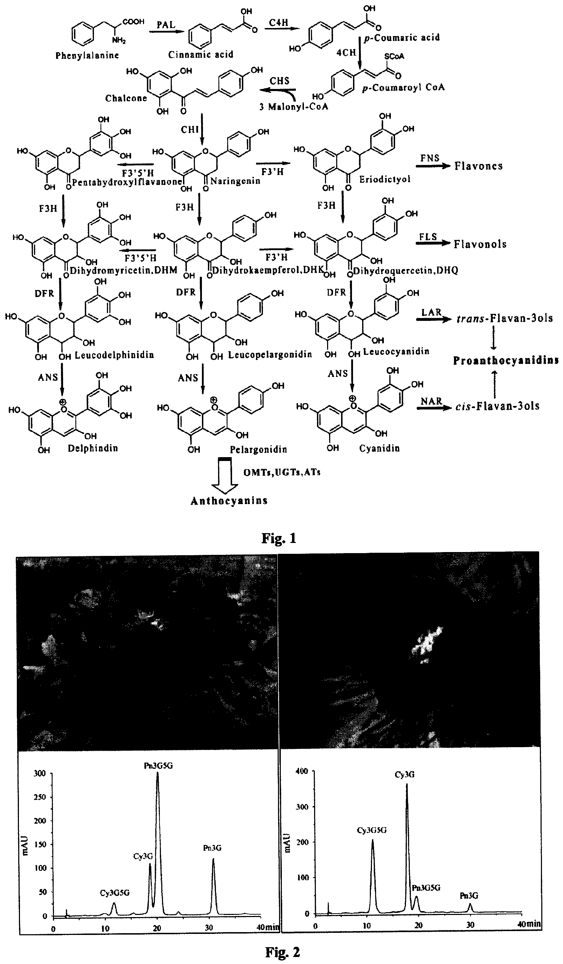

Flavonoids is a large group of secondary metabolites in plant, including anthocyanin, flavone, flavonol, flavan, flavanol and isoflavone etc., which provide pigmentation, protection against UV photo-damage by anthocyanin and other flavonoid as copigment, structural support by polymeric lignin and assorted antimicrobial phytoalexins. These compounds are derived from the primary metabolism of phenylalanine. The entry point of the flavonoid biosynthesis is cinnamic acid, which is produced by the first reaction of phenylalanine with phenylalanine ammonia-lyase (PAL, Fig. 1). Following, a hydroxyl group is introduced at the *para* position of the phenyl ring of cinnamic acid catalyzed by cinnamic acid 4-hydroxylase (C4H), producing *p*-coumaric acid. The carboxyl group of *p*-coumaric acid is then activated by formation of a thioester bond with CoA, which is a process catalyzed by *p*-coumaroyl:CoA ligase (4CL). Three malonyl-CoA are condensed iteratively onto the end of one *p*-coumaric acid CoA catalyzed by chalcone synthase (CHS), producing chalcone. Chalcone isomerase (CHI) stereospecifically directs and greatly accelerates the spontaneous additional cyclization of chalcones to form naringenin. Flavanone 3-hydroxylase (F3H), flavanone 3'-hydroxylase (F3'H), and flavanone 3',5'-hydroxylase (F3'5'H) subsequently hydroxylate naringenin at 3-, 3'-, 3'5'-position, forming dihydroflavonol (DHK, DHQ, DHM, respectively) (Vogt, T., Phenylpropanoid biosynthesis. Molecular Plant, 2010. 3(1): p. 2-20.). Dihydroflavonol 4-reductase (DFR) converts dihydroflavonols into flavandiols (leucoanthocyanidin) by reducing the ketone group on the central ring. The leucoanthocyanidins are converted into three corresponding anthocyanidins (pelargonidin, cyanidin and delphinidin) by the action of an anthocyanidin synthase (ANS)/leucoanthocyanidin dioxygenase (LDOX). Except for the three major anthocyanidins, more than 14 anthocyanidins are described (Grotewold, E., The genetics and biochemistry of floral pigments. Annual Review of Plant Biology, 2006. 57: p. 761-780.). Though thousands of anthocyanins have been reported, they mostly derive from six common anthocyanidins: pelargonidin (Pg), cyanidin (Cy), delphinidin (Dp), peonidin (Pn), petunidin (Pt) and malvidin (Mv). In terms of biosynthesis pathway, peonidin is of mono-methylated cyanidin at 3'- position of B-ring, petunidin and malvidin are both derived from delphinidin with one or two methyl groups at 3'- and 3'5'- position of B-ring. Numerous anthocyanins are derived from the six anthocyanidins with modifications by glycosylation, methylation, and acylation.

Chromophore of anthocyanin is mainly aglycone, delphinidin and its derivates tend to have blue color, and pelargonidin derivates contribute to intense red color. Methylation of 3'- or 5'-hydroxyl group results in a slight reddening (Tanaka, Y., F. Brugliera, and S. Chandler, Recent progress of flower colour modification by biotechnology. International Journal of Molecular Sciences, 2009. 10(12): p. 5350-5369.). Though glycosylation and acylation don't change the absorption wavelength, they facilitate stabilization, and even turn red anthocyanins to blue by acylation packaging (Shiono, M., N. Matsugaki, and K. Takeda, Structure of the blue cornflower pigment - packaging red-rose anthocyanin as part of a 'superpigment' in another flower turns it brilliant blue. Nature, 2005. 436(7052): p. 791-791).

Other flavonoids such as flavone, flavonol, isoflavone are synthesized by flavone synthase (FNS), flavonol synthase (FLS), and isoflavone synthase (IFS), respectively. Their derivates undergo similar modifications. In particular, *O*-methylation of hydroxyl groups in flavonoids reduces their reactivity and increases their antimicrobial activity (Ibrahim, R.K., et al., Enzymology and compartmentation of polymethylated flavonol glucosides in Chrysosplenium-Americanum. Phytochemistry, 1987. 26(5): p. 1237-1245.).

Flower color formation is due to three types of chemically distinct pigment: flavonoids, betalains, and carotenoids among them, flavonoids have been the most extensively studied for their broader distribution among the angiosperms. The flavonoid molecules that make major contribution to flower color are anthocyanins, which are responsible for the majority of the orange, red, purple, and blue colors of flowers. The flavonoids other than anthocyanins can serve as copigment that affect the flower coloration, such as flavones and flavonols (Figueiredo, P., et al., New aspects of anthocyanin complexation. Intramolecular copigmentation as a means for colour loss? Phytochemistry, 1996. 41(1): p. 301-308.). In addition, some other factors influence the hue of flower. For instance, vacuolar pH plays an important role in hue of anthocyanins that are located in the vacuole of epidermal cell of flower petal (Fukada-Tanaka, S., et al., Colour-enhancing protein in blue petals - Spectacular morning glory blooms rely on a behind-the-scenes proton exchanger. Nature, 2000. 407(6804): p. 581-581.). In addition to pH, metal ion (Kondo, T., et al., Structural basis of blue-color development in flower petals from Commelina communis. Nature, 1992. 358(6386): p. 515-518.), the stacking of anthocyanins (anthocyanic vacuolar inclusion, AVI) (Pourcel, L., et al., The formation of anthocyanic vacuolar inclusions in Arabidopsis thaliana and implications for the sequestration of anthocyanin pigments. Molecular Plant, 2010. 3(1): p. 78-90.), and cell shape (Noda, K., et al., Flower color intensity depends on specialized cell-shape controlled by a MYB-related transcription factor. Nature, 1994. 369(6482): p. 661-664.) also have a dramatic impact on hue changes.

Ornamental plants play an important aesthetic role in decoration of human life by providing a broad range of colors. Although increasing postharvest life, altering scent, and modifying flower shape are aims that are pursuing, altering specific color traits to generate novel color ornamental plant is the major goal of breeding (Tanaka, Y., et al., Genetic engineering in floriculture. Plant Cell Tissue and Organ Culture, 2005. 80(1): p. 1-24.). Anthocyanins in particular have also been the target of numerous biotechnological efforts with the objective of creating new, or altering the properties of existing and pigment compounds (Grotewold, E., The genetics and biochemistry of floral pigments. Annual Review of Plant Biology, 2006. 57: p. 761-780.). These three essential anthocyanidins (Pg, Cy and Dp) synthesis is shown in Fig. 1, however, the question of how the other three common anthocyanidins (Pn, Pt, Mv) are synthesized is crucial and unclear. The investigation on the formation of methylated anthocyanidins since 1977, Wiering and Devlaming showed that there were two pairs of duplicate genes controlling methylation of anthocyanin in petunia, (Wiering, H. and P. Devlaming, Glycosylation and methylation patterns of anthocyanins in Petunia-hybrida .2. Genes Mf1 and Mf2. Zeitschrift Fur Pflanzenzuchtung-Journal of Plant Breeding, 1977. 78(2): p. 113-123.), showing activity with cyanidin 3-(*p*-coumaroyl)-rutinoside-5-glucoside, delphinidin 3-(*p*-coumaroyl)-rutinoside-5-glucoside *in vitro* (Jonsson, L.M.V., et al., Properties and genetic-control of 4 methyltransferases involved in methylation of anthocyanins in flowers of petunia-hybrida. Planta, 1984. 160(2): p. 174-179.), however, the encoding gene in petunia have not been cloned to date. A SAM and Mg²⁺ dependent cyanidin 3-glucoside 3'-*O*-methyltransferase (CGMT) was partially purified from an anthocyanin-promoting cell suspension of *Vitis vinifera* (Bailly, C., F. Cormier, and C.B. Do, Characterization and activities of S-adenosyl-L-methionine:cyanidin 3-glucoside 3'-O-methyltransferase in relation to anthocyanin accumulation in Vitis vinifera cell suspension cultures. Plant Science, 1997. 122(1): p. 81-89.), the activity of CGMT was 13% using cyanidin as substrate, no activity with cyanidin 3-*p*-coumaroyl glucoside suggesting that methylation occurred before acylation, and no activity with delphinidin, indicating that more than one OMT involved in anthocyanin synthesis in grape. Hugueney et al. isolated a novel cDNA encoding anthocyanin *O*-methyltransferase (AOMT) in grapevine, and validated its 3',5'-*O*-methyltransferase activity *in vitro* and *in planta* (Hugueney, P., et al., A novel cation-dependent O-methyltransferase involved in anthocyanin methylation in grapevine. Plant Physiology, 2009. 150(4): p. 2057-2070.). A gene from another grapevine cultivar having same open reading frame (ORF) with *VvAOMT* also was analyzed by Lucker et al., showing 3',5'-*O*-methyltransferase with strong preference for anthocyanins and glycosylated flavonols *in vitro* (Lucker, J., S. Martens, and S.T. Lund, Characterization of a Vitis vinifera cv. Cabernet Sauvignon 3,5'-O-methyltransferase showing strong preference for anthocyanins and glycosylated flavonols. Phytochemistry, 2010. 71(13): p. 1474-1484.). Brugliera et al. revealed two flavonoid methyltransferase genes from petunia (*FMT*) and torenia (*TFMT*), and their crude expressed protein in *E. coli* were able to catalyze delphinidin 3-rutinoside and delphinidin 3-glucoside to corresponding petunidin or malvidin derivates. The antisense expression of *FMT* in petunia plants showed that the petal color was changed from purple in control to dark pink or red purple in separate transgenic petunia flower (BRUGLIERA, F., et al., *Genetic sequences having methyltransferase activity and uses therefor.* 2003, WO Patent 2,003,062,428.). The gene *CkmOMT2* from purple-flowered fragrant cyclamen 'Kaori-no-mai' (KM), and enzyme assay *in vitro* demonstrated that CkmOMT2 was responsible for the methylation of 3' or 3',5'-*O*- of anthocyanin substrates. A deletion of *CkmOMT2* gene on genome by ion-beam irradiation induced a red-purple flowered fragrant cyclamen (KMrp) (Akita, Y., et al., Isolation and characterization of the fragrant cyclamen O-methyltransferase involved in flower coloration. Planta, 2011. 234(6): p. 1127-1136.). It indicated that methylation of anthocyanin contributes to flower color variation.

A method of controlling the activity of anthocyanin methyltransferase would provide a means of manipulating flower color, therefore enabling a single species or cultivar to possess a broader range of petal colors. Such control may be achieved by regulating the expression level or activity of an indigenous enzyme, or by introducing a non-indigenous enzyme.

### SUMMARY OF THE INVENTION

The present invention provides the following:
(1) A protein having an amino acid sequence shown in SEQ ID NO: 3, or an amino acid sequence having deletion, substitution or insertion of one or plural amino acids in said amino acid sequence.
(2) The said protein, which is characterized in that it has anthocyanin 3'-*O*-methyltransferase or 3',5'-*O*-methyltrasnferase activity.
(3) A gene comprising the nucleotide sequence shown in SEQ ID NO: 1, or a gene which hybridizes with said gene under stringent conditions and encodes a protein, which has anthocyanin 3'-*O*-methyltransferase or 3',5'-*O*-methyltrasnferase activity.
(4) A gene encoding the above-mentioned protein.
(5) An expression vector comprising the above-mentioned gene.
(6) A transformant transformed with the said expression vector.
(7) A partial peptide of the above-mentioned protein.
(8) A method for obtaining the transgenic organism, which is characterized in that the above-mentioned gene is used.
(9) A transgenic organism, wherein the above-mentioned gene is artificially introduced into the target organism.
(10) A method to manipulate the activity of the above-mentioned protein, which is characterized in the mutation of the single amino acid which is crucial for catalytic reaction, is carried out.

The present invention relates to a genetic sequence encoding a flavonoid *O*-methyltransferase (PsFOMT) from tree peony, and a method to modulate the activity of PsFOMT by single amino acid replacement which is crucial for catalytic reaction. In China, tree peony (*Paeonia spp*.; Chinese Mudan) has been named the "king of flowers", bringing good fortune and happiness. Wang et al. analyzed 130 Zhongyuan and 37 Daikon Island tree peony cultivars, and revealed that there were six anthocyanins: Pn3G5G, Pn3G, Cy3G5G, Cy3G, Pg3G5G and Pg3G (Wang, L.S., et al., Analysis of petal anthocyanins to investigate flower coloration of Zhongyuan (Chinese) and Daikon Island (Japanese) tree peony cultivars. Journal of Plant Research, 2001. 114(1113): p. 33-43.), and all accessions contained Pn3G5G as a dominant anthocyanin (Wang, L.S., et al., Phenetics in tree peony species from China by flower pigment cluster analysis. Journal of Plant Research, 2001. 114(1115): p. 213-221.). The inventors surprisingly noticed that hundreds of tree peony cultivars have purple or purplish flower color and the main anthocyanins in those cultivars flower petals are Pn3G5G, which contributed to the decrease of b* value (CIELAB system, International Commission on Illumination), it is indicated that the petal hue turn to purple while the content of Pn3G5G increase. To explain the color formation mechanism, the inventors searched tree peony cultivars and their relative herbaceous peony *Paeonia tenuifolia* for a control, which has different flower colors, while, in vivid red petals, Cy3G was the main anthocyanin composition (Fig. 2). A small molecular type I *O*-methyltransferase gene (*PsFOMT*) was isolated from Japanese tree peony cultivar *Paeonia suffruticosa* cv. 'Gunpohden' and the enzyme efficiently act on flavonoid in particular anthocyanins, turned out to be F3'OMT and F3'5'OMT. From the counterpart *Paeonia tenuifolia,* we cloned the similar gene *PtFOMT*, which has four nucleotides different from *PsFOMT* and whose encoding enzyme has a significant decrease in activity. The four different nucleotides were corresponded to four various amino acid residues. The role of each residue was confirmed by site-directed mutagenesis, revealing that only one residue was responsible for the catalytic activity, which can be a target to modulate the methylation in plant. The invention includes an anthocyanin OMT and the method to decrease OMT activity, provide an optional approach to modify plant characteristic such as flower, fruit, leave, seed color etc., and develop new cultivars with altered color feature, and engineering bacteria with specific function. It also can be used in other aspects, for instance, the novel extract of FOMT transformed plants could be used as a healthcare additive, or beverage, juice, food coloring. Beverages may include but are not limited to wine, tea, coffee, milk, and dairy products.

### BRIEF DESCRIPTION OF THE DRAWINGS

Fig.1 Schematic representation of the biosynthetic pathway of flavonoids. The name and structure of compounds are indicated. The enzyme names are PAL, phenylanine ammonialyase; C4H, cinnamate 4-hydroxylase; 4CH, 4-coumarate: CoA ligase; CHS, chalcone synthase; CHI, chalcone isomerase; F3H, flavanone 3-hydroxylase; F3'H, flavanone 3'-hydroxylase; F3'5'H, flavanone 3',5'-hydroxylase; DFR, dihydroflavonol 4-reductase; ANS, anthocyanidin synthase; FNS, flavones synthase; FLS, flavonol synthase; LAR, leucoanthocyanidin reductase; ANR anthocyanidin reductase; OMT, *O*-methyltransferase; UGT, UDP-glucose: flavonoid glucosyltransferase; AT, acyltransferase. The virtual tail arrow part needs to be refined and validated.
Fig. 2 The flower images and anthocyanin profiles. A, *Paeonia suffruticosa* cv. 'Gunpohden', with purple color; B, *Paeonia tenuifolia* with vivid red color.
Fig. 3 Diagrammatic representation of expression plasmids in *E. coli* (pMAL-PsFOMT, pMAL-PtFOMT), and binary plasmids for *Agrobacterium*-mediated transformation (pBI121-PAP1, and pBI121-PsFOMT, pBI121-PtFOMT).
Fig. 4 The comparison of PsFOMT and PtFOMT polypeptide, and the four amino acid mutations (positions 13, 85, 87, and 205).
Fig. 5 The amino acid sequence of PsFOMT was aligned with VvAOMT (grapevine), GmAOMT (black soybean), MsPFOMT (ice plant), and its secondary structure was predicted compared with 3C3Y_A by LOOPP program package.

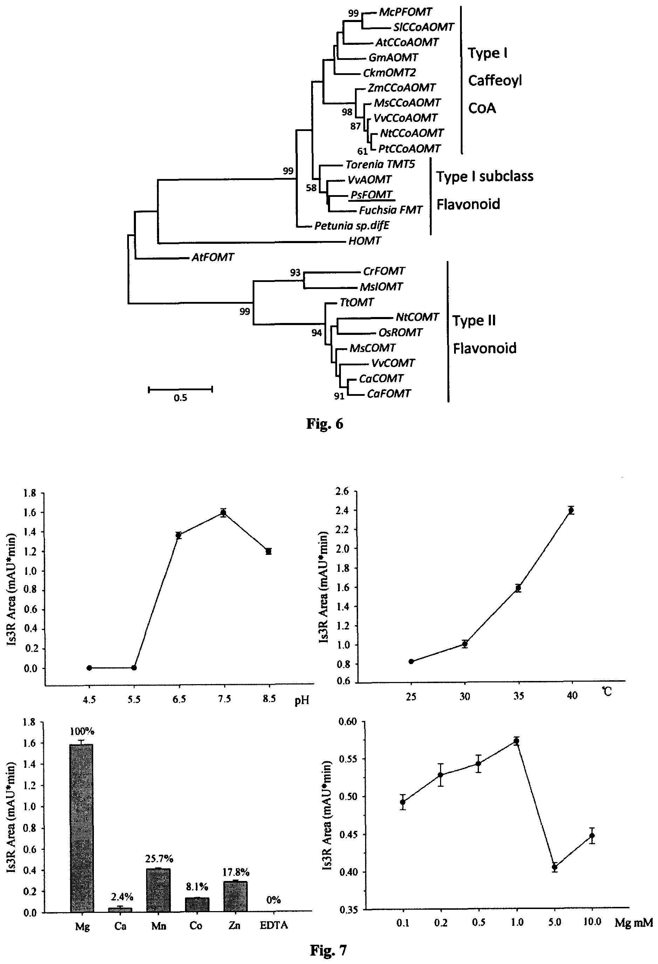

Fig. 6 Phylogenetic tree of selected OMT amino acid sequences. Type I OMTs are in the upper clade, including caffeoyl-CoA OMT from *Arabidopsis thaliana* (*AtCCoAOMT*; accession no. AAM64800), *Stellaria longipes* (*SlCCoAOMT*; L22203), *Medicago sativa* (*MsCCoAOMT*; AAC28973), *Nicotiana tabacum* (*NtCCoAOMT*; U38612), *Vitis vinifera* (*VvCCoAOMT*; Z54233), *Populus balsamifera* subsp. (*PtCCoAOMT*; AJ224896), *Zea mays* (*ZmCCoAOMT*; AJ242980), catechol OMT from *Homo sapiens* (*HOMT*; A38459), *Thalictrum tuberosum* (*TtOMT*; AF064693). Type I subclass OMTs in boldface were the ones reported so far that have methylation activity for anthocyanins, including anthocyanin OMT from *Vitis vinifera* (*VvAOMT*; BQ796057), *Glycine max* (*GmAOMT*; ADX43927.1), *Cyclamen persicum* × *Cyclamen purpurascens* (*CkmOMT2*; BAK74804.1), *Fuchsia* (*FMT*; HB975539.1), *Torenia sp.* (*TMT5*; HB975529.1), *Petunia sp.* (*Petunia difE FMT*; HB975519.1). Type II include caffeic acid OMT from *Chrysosplenium americanum* (*CaCOMT*; AAA86982), *Medicago sativa* (*MsCOMT*; M63853), *Vitis vinifera* (*VvCOMT*, AF239740), *Nicotiana tabacum* (*NtCOMT*; AF484252), flavonoid OMT from *Arabidopsis thaliana* (*AtFOMT*; AY087244), *Mesembryanthemum crystallinum* (*McPFOMT*; AY145521), *Chrysosplenium americanum* (*CaFOMT*; U16794), *Catharanthus roseus* (*CrFOMT*; AY127568), *Oryza sativa* (*OsROMT*; DQ288259), and isoflavone OMT from *Medicago sativa* (*MsIOMT*; U97125). The number beside the branches stands for the bootstrap values based on 1000 replicates using Mega 5.0.
Fig. 7 The activity of PsFOMT *in vitro* with different reaction systems, including pH of buffer solution, incubation temperature, the presence of different divalent cations, and the concentration of Mg²⁺. Results are means of triplicate experiments.
Fig. 8 Representation of the product increase within 6 minutes in the presence of PsFOMT with Cy3G as substrate. The product was identified with Pn3G as standard reference.
Fig. 9 Characterization of PsFOMT activity in planta. A, transgenic tobacco flowers with empty vector control; B, strawberry fruits with transient expression of *PAP1* and coexpression of *PAP1* and *PsFOMT.*
Fig. 10 The transgenic tobacco flowers with empty vector (pink color) and 35S::*PsFOMT* cDNA (blueish pink color), respectively.
Fig. 11 Accumulation of anthocyanins in developmental flowers of *Paeonia suffruticosa* cv. 'Gunpohden', and expression profiling of *PsFOMT* with control of constitutively expressed genes *Actin.* The horizontal coordinate stands for the date of sample collection.

### DETAILED DESCRIPTION OF THE INVENTION

The present invention will be explained in detail below.

The present invention relates to a genetic sequence encoding a SAM-dependent flavonoid *O*-methyltransferases (FOMT) which use anthocyanins as optimum substrates, and a core amino acid site which is vital for the catalytic activity, and their uses and/or the corresponding polypeptide thereof. More particularly, the invention relates to a polypeptide which has anthocyanin 3'-*O*-methyltransferase or 3',5'-*O*-methyltrasnferase activity. Cyanidin, delphinidin, and quercetin glycosides can be used as substrates. The function of *FOMT* gene was validated by recombinant protein enzymatic assay *in vitro* and transgenic pant analysis *in vivo.* Moreover, a method to manipulate the activity of this polypeptide was revealed. There is a single amino acid that controls the activity on the polypeptide, which is a target to modify and regulate the methylation of anthocyanin. The invention provides an access to manipulate phenotypes of a plant related to flavonoids constitute, such as coloration and antioxidation. The invention further relates to sense and antisense sequences to all or part of the gene as well as the transgenic plants and their reproductive tissues.

### EXAMPLE 1

### Plant material

Two accessions of Paeoniaceae, Paeonia (*Paeonia suffruticosa* cv. 'Gunpohden' and *Paeonia tenuifolia*) were used as subjects, which have distinct flower color phenotypes, being purple or vivid red, respectively. The flower petals of different developing stages were collected for flavonoids profile, gene cloning and expression analysis.

### Bacterial strains

DH5α, F- supE44, Δ(lacZYA-ArgF)U169,(Φ80lacZΔM15) hsdR17(rₖ⁻,mₖ⁺) recA1 endA1 gyrA96 relA1 deoR; BL21, F- dcm ompT hsdS(rB- mB-) gal araB::T7RNAP-tetA was used. The *Agrobacterium tumedaciens* strain EHA105 was used (Saved by our lab, and disclosed in the non-patent document: Gao Shiwu, et al., Factors affecting transformation efficiency of Agrobacterium tumefaciens EHA105 competent cells, Journal of South China University of Tropical Agriculture, 2012, 3(1)).

### General methods

In general, the methods followed were as described in Shambrook et al. (Molecular Clonging: A laboratory manual. (3rd ed.), Cold Spring Harbor Laboratory Press, USA, 2001.). The cloning vector pEASY-T3 was obtained from TransGen Biotech, the bacterial expression vector pMAL-c5X (Purchased from NEB) and eukaryotic expression vector pBI121 (Saved by our lab) were donated by colleague.

### Cloning of FOMTs

Total RNA from petal of two accessions were isolated using a TIANGEN RNA Isolation Kit (TIANGEN, Beijing, China). To remove contaminating DNA, the total RNA was treated with 10 units of RNase-free DNase1 (Takara, Japan) for 30 min at 37°C, then inactivated DNase1 in 65°C for 10 min. Final RNA concentration was determined using Nanodrop 2000 (Thermo). RNA was converted into cDNA using M-MLV Reverse Transcriptase (Promega, USA). To identify candidate genes, we searched the tree peony flower bud EST database (Shu, Q.Y., et al., Functional annotation of expressed sequence tags as a tool to understand the molecular mechanism controlling flower bud development in tree peony. Physiologia Plantarum, 2009. 135(4): p. 436-449.), the primers used to obtain open reading frame (ORF) are shown in Table 1. A high fidelity polymerase (Takara, Japan) was used for PCR, and the program was as follows: 95°C for 5 min, then 30 cycles of 95°C for 30 s, 55°C for 30 s, and 72°C for 90 s; followed by elongation at 72°C for 10 min. The PCR product was separated by agarose gel electrophoresis. The DNA fragment of interest was purified and recovered by EasyPure Quick Gel Extraction Kit (TransGen, China) according to manufacturer's instructions. Then the DNA fragment of interest was ligated into the pEASY-T3 vector (Takara, Japan) for sequencing. The isoelectric point of FOMTs was predicted by Compute pI/MW from ExPASy (http://www.expasy.ch/tools/). Tertiary and quaternary structure of FOMT proteins from sequence were predicted by PyMOL software.

**Table 1 Summary of sequence identities**

| SEQ ID No. | Name | Description |
|---|---|---|
| 1 | *PsFOMT* | cDNA nucleotide |
| 2 | *PtFOMT* | cDNA nucleotide |
| 3 | PsFOMT | cDNA amino acid |
| 4 | PtFOMT | cDNA amino acid |
| 5 | *Bam*HI-forward | oligonucleotide |
| 6 | *Xho*I-reverse | oligonucleotide |
| 7 | *Nde*I-forward | oligonucleotide |
| 8 | *Bam*HI-reverse | oligonucleotide |
| 9 | *PtFOMT* G13E-forward | oligonucleotide |
| 10 | *PtFOMT* G13E-reverse | oligonucleotide |
| 11 | *PtFOMT* T85A-forward | oligonucleotide |
| 12 | *PtFOMT* T85A-reverse | oligonucleotide |
| 13 | *PtFOMT* R87L-forward | oligonucleotide |
| 14 | *PtFOMT* R87L-reverse | oligonucleotide |
| 15 | *PtFOMT* T205R-forward | oligonucleotide |
| 16 | *PtFOMT* T205R-reverse | oligonucleotide |
| 17 | *PsFOMT* L87R-forward | oligonucleotide |
| 18 | *PsFOMT* L87R -reverse | oligonucleotide |
| 19 | *PsFOMT* L87A-forward | oligonucleotide |
| 20 | *PsFOMT* L87A-reverse | oligonucleotide |
| 21 | *PsFOMT*-forward | oligonucleotide |
| 22 | *PsFOMT*-reverse | oligonucleotide |

### Analysis of PsFOMT gene expression during flower development

Flower petals of *Paeonia suffruticosa* cv. 'Gunpohden' at five developmental phase (from colorless, to blossom) were collected once every three days for RNA extraction. The mRNA sample was purified and converted into cDNA. The semi quantitative RT-PCR was performed in triplicates with specific primer SEQ No. 21 & 22. PCR was carried out for 25 cycles of denaturation at 94°C for 30 s, annealing at 56°C for 30 s, and extension at 72°C for 60 s. The PCR products were separated on an agarose gel. The constitutively expressed *actin* gene was used as a control for expression analysis.

### Expression and purification of recombinant FOMTs

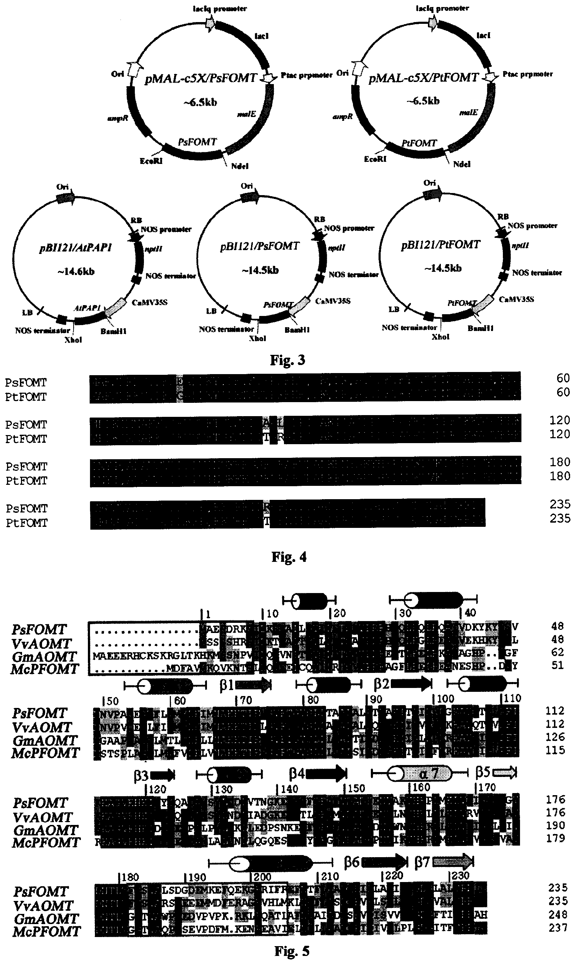

Full length *FOMTs* were amplified by primers SEQ No.7 & 8 in which restriction enzyme *Nde*I and *Bam*HI site were introduced to the 5'-end and 3'-end, respectively. The DNA fragment of interest was ligated into the pEASY-T3 vector, and then the recombinant plasmid was transducted into DH5α. The FOMT cDNAs were sequenced to verify that no mutation occurred. The recombinant plasmid was isolated and digested with *Nde1* and *Bam*HI. Subsequently, the fragment was ligated to the *Nde*I and *Bam*HI excised expression vector pMAL-c5X with a MBP tag to yield pMAL-PsFOMT and pMAL-PtFOMT (Fig. 3). The correct construction tested by sequencing was introduced into *E. coli* strain BL21. *E. coli* harboring pMAL-PsFOMT and pMAL-PtFOMT were cultivated in 2 mL LB liquid medium containing glucose, supplemented by 100 µg/mL ampicillin until OD₆₀₀ reached 0.4 - 0.5. After addition of isopropyl *β*-D-thiogalactopyranoside (IPTG) to a final concentration of 0.1 mM, the cells were further cultured at 16°C for 20 h. Harvest the cells by centrifugation and discard the supernatant. The cells were resuspended in column buffer (20 mM Tris-HCl, 200 mM NaCl 1 mM EDTA, 10 mM *β*-mercaptoethanol, pH 7.4), and disrupted by sonication in an ice-water bath (Lan, T., et al., Extensive functional diversification of the Populus glutathione S-transferase supergene family. Plant Cell, 2009. 21(12): p. 3749-3766.). In each case, the homogenate was then subjected to centrifugation at 10,000g for 10 min at 4°C. The resultant particulate material and a small portion of the supernatant were analyzed by SDS-PAGE. The rest of the supernatant was loaded onto an amylose resin column (NEB) that had been pre-equilibrated with column buffer. The overexpressed fusion protein bound to the column was eluted with column buffer plus 10 mM maltose. The concentration of purified fusion protein was detected by UV absorbance at 280 nm. As a control, BL21 cells transformed with an empty pMAL-c5X vector were assayed. The purified protein was stored at -80°C mixed with 10% glycerol.

### Biochemical Characterization of recombinant FOMTs

For quantitative analyses, reaction conditions were optimized. The pH dependence of FOMTs activity was assessed in the pH range of 4.5 to 8.5 using MES (4.5 - 6.5) and Tris-HCl (7.5 - 8.5) buffer. The effect of divalent cations on enzyme activity was estimated by adding to the reaction mixture containing 10 mM MgCl₂, CaCl₂, ZnCl₂, MnCl₂, CoCl₂ or EDTA. The proper concentration of metal ion was assessed by testing different concentrations of MgCl₂ (0.1, 0.2, 0.5, 1.0, 5.0 and 10 mM). Incubation temperature was set at 25, 30, 35 and 40°C to detect the effect on enzyme activity.

The reaction system was according to Hugueney et al. with some modification (Hugueney, P., et al., A novel cation-dependent O-methyltransferase involved in anthocyanin methylation in grapevine. Plant Physiology, 2009. 150(4): p. 2057-2070.). Purified recombinant FOMT (2 µg) was assayed in a final volume of 200 µL containing 200 µM SAM, 1 mM MgCl₂, 14 mM *β*-mercaptoethanol, 100 mM Tris, pH 7.5, and 20 µM flavonoid substrates. Incubation was performed at 35°C and stopped with 200 µL methanol containing 2% formic acid. For enzyme kinetic studies, purified FOMT was incubated based on the above condition with the exception that a range of substrate concentrations from 5 to 200 µM were used for *Kₘ* determination. Reaction product was analyzed by a Dionex HPLC system (Sunnyvale, CA), equipped with a P680 HPLC pump, an UltiMate 3000 autosampler, a TCC-100 thermostated column compartment and a Dionex PDA100 photodiode array detector. The analytical column was C₁₈ column of ODS 80Ts QA (150 mm × 4.6 mm, 5 µm i.d., Tosoh, Tokyo) protected with a C₁₈ guard cartridge (Shanghai ANPEL Scientific Instrument, Shanghai). The following solvent and gradient were used: A, 10% aqueous formic acid; B, methanol; constant gradient from 10 to 36% B within 15 min and back to 10% B in 3 min; the flow rate was 0.8 mL min⁻¹; Column temperature was maintained at 35°C; 20 µL of analyte was injected. Chromatograms were obtained at 525 nm for anthocyanins and 350 nm for other flavonoids, and photodiode array spectra were recorded from 200 to 800 nm. *Kₘ* and *Vₘₐₓ* values were calculated from Lineweaver-Burk plots.

### Result

### Sequences analysis of PsFOMT and PtFOMT

The cDNA ORF sequence and the putative translated amino acid sequence of *PsFOMT* and *PtFOMT* (SEQ1 to SEQ4) were shown in Table 1. The cDNA sequence length is 708 bp and the putative amino acid sequence is 235 aa. The theoretical pI of PsFOMT and PtFOMT were 5.25 and 5.36, respectively, and both Mw were 26.4 kD. The comparison analysis of the two amino acid sequences was performed (Fig. 4), showing four differences at positions of 13, 85, 87, and 205. The amino acid sequence of PsFOMT was aligned with VvAOMT (grapevine), GmAOMT (black soybean) and MsPFOMT (ice plant). PsFOMT presented 72% and 69% identity with VaAOMT and MsPFOMT, respectively. The secondary structure of PsFOMT was predicted compared with 3C3Y A by LOOPP program package, which contained a conserved domain (Pfam01596) and a structure of 8 *α*-helixes and 7 *β*-sheets (Fig. 5).

### Dendogram of plant OMTs

The homologous genes from various species were compared using a BLAST search in NCBI (www.ncbi.nlm.nih.gov). Nucleic acid and amino acid sequences were aligned using CLUSTAL X (Thompson, J.D., et al., The CLUSTAL_X windows interface: flexible strategies for multiple sequence alignment aided by quality analysis tools. Nucleic Acids Research, 1997. 25(24): p. 4876-4882.) and refined manually. MEGA 5.0 software was used to construct a phylogeny tree using the maximum likelihood test method (Tamura, K., et al., MEGA5: Molecular evolutionary genetics analysis using maximum likelihood, evolutionary distance, and maximum parsimony methods. Molecular Biology and Evolution, 2011. 28(10): p. 2731-2739.), with 1000 bootstrap replicates. Phylogenetic analysis showed that *PsFOMT* belongs to a subclade of type I OMTs, closely related to the anthocyanin-OMT *VvAOMT* from grapevine, flavonoid-OMTs from petunia difE, torenia, and fuchsia (incomplete ORF), and *PFOMT* from *M. crystallinum* and *AtCCoAOMT* from Arabidopsis (Fig. 6).

### Flavonoid O-methyltransferases activity of recombinant protein

The pH dependence of PsFOMT *in vitro* was in a wide range of 6.5 - 8.5, with an optimum of 7.5 on the substrate of Qu3R. With the increase of incubation temperature (25 - 40°C), the activity of PsFOMT was accelerated. The influence of different divalent cations was tested, and the results showed that PsFOMT was the most active in the presence of Mg²⁺. The optimal concentration of Mg²⁺ was 1.0 mM. The activity of PsFOMT could not been detected in the presence of EDTA (Fig. 7). It is indicated that PsFOMT is an Mg²⁺-dependent enzyme, which is consistent with the prediction of type I OMT.

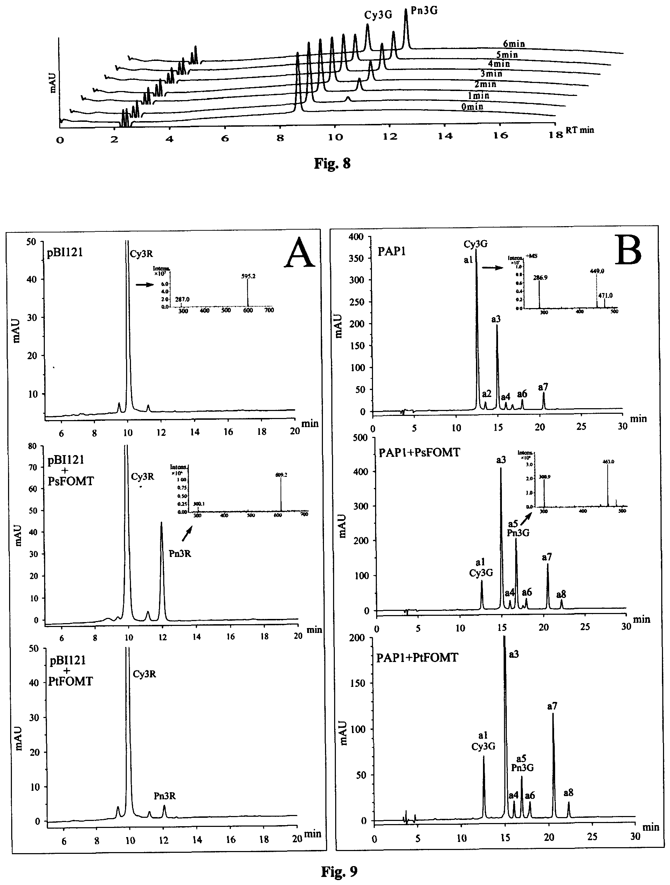

The enzyme activity of purified fusion protein (PsFOMT and PtFOMT) were assessed *in vitro* using substrates including cyanidin, delphinidin , quercetin, pelargonidin 3-*O*-glucoside, cyanidin 3,5-di-*O*-glucoside, cyanidin 3-*O*-glucoside, cyanidin 3-*O*-galactoside, delphinidin 3-*O*-glucoside, quercetin 3-*O*-rutinoside, caffeic acid, luteolin, kaempferol andnaringenin, epicatechin, in the presence of SAM. The PsFOMT fusion protein has high catalytical efficiency specific for cyanidin 3,5-di-*O*-glucoside, cyanidin 3-*O*-glucoside. It also can sequentially methylate 3'- and 5'-OH at B ring of delphinidin 3-*O*-glucoside. Cyanidin 3-*O*-galactoside, quercetin 3-*O*-rutinoside and quercetin can be methylated by PsFOMT at different level (Table 2). The reaction product increased within 6 minutes in the presence of PsFOMT with Cy3G as substrate (Fig. 8). The product was identified with Pn3G as standard reference. The PtFOMT protein which is extremely similar with PsFOMT has a low methyltransferase activity for the substrates tested (Table 3). It is suggested that the four variant amino acids might be responsible for the divarication of enzyme activity between PsFOMT and PtFOMT.

**Table 2 Kinetic parameters of PsFOMT with potential substrates.**

| Substrate | *K*ₘ *µM* | *V*ₘₐₓ *nM s⁻¹* | *K*_{cat} × 10 *s⁻¹* | *K*_{cat}/*K*ₘ *M⁻¹s⁻¹* | Specific Activity *pkat mg⁻¹* |
|---|---|---|---|---|---|
| Pelargonidin 3-*O*-glucoside | - | - | - | - | - |
| Cyanidin 3,5-di-*O*-glucoside | 1.06 (0.01) | 17.88 (0.25) | 127.70 (1.05) | 120886 (563) | 1788 (15) |
| Cyanidin 3-*O*-glucoside | 1.76 (0.02) | 11.57 (0.05) | 161.99 (0.73) | 91829 (421) | 2314 (11) |
| Cyanidin 3-*O*-galactoside | 31.69 (2.07) | 39.22 (6.57) | 217.90 (36.48) | 6981 (1311) | 3138 (525) |
| Delphinidin 3-*O*-glucoside | 4.11 (0.35) | 17.05 (0.27) | 94.72 (1.49) | 23293 (1538) | 1364 (21) |
| Quercetin 3-*O*-rutinoside | 12.32 (0.32) | 27.44 (1.63) | 192.11 (4.41) | 15599 (110) | 2744 (135) |
| Cyanidin | - | - | - | - | - |
| Delphinidin | - | - | - | - | - |
| Quercetin | 4.33 (0.07) | 1.77 (0.02) | 9.83 (0.12) | 2271 (24) | 142 (2) |
| Caffeic acid | - | - | - | - | - |
| Luteolin | - | - | - | - | - |
| Kaempferol | - | - | - | - | - |
| Naringenin | - | - | - | - | - |
| Epicatechin | - | - | - | - | - |

| | | | | | |
|---|---|---|---|---|---|
| Data are expressed as means (SE) of triplicate assays. -, no product detected | | | | | |

**Table 3 Enzymatic activity of PtFOMT with potential substrates.**

| Substrate | Specific activity *pkat mg⁻¹* |
|---|---|
| Cy3G | 37.98 ± 1.05 |
| Cy3G5G | 29.60 ± 3.47 |
| Dp3G | 21.50 ± 0.10 |
| Qu3R | 30.20 ± 0.24 |
| Qu | 17.58 ± 0.57 |

| | |
|---|---|
| Data are expressed as means ± SE of triplicate assays. | |

### EXAMPLE 2

### Characterization of PsFOMT activity in plant

The full length genes were introduced with a *Bam*H1 site and an *XhoI* site on 5'- and 3'-end by PCR with primers SEQ No. 5 & 6. The double digested fragment of interest and eukaryotic expression vector pBI121 with *Bam*H1 site and an *Xho*I were ligated. Then the appropriate constructs were introduced into *Agrobacterium* strain EHA105 by electroporation.

### Transgenic tobacco

Leaf of *Nicotiana tabacum* cv.Nc89 plant (Donated by Professor Silan Dai, Beijing Forestry University) was disinfected by 75% ethanol for 30 s, followed by 2% sodium hypochlorite for 3 minutes, and then washed with sterile water for three times. The leaf was cut into squares (25 mm²). A single colony of *Agrobacterium* strain EHA105 with pBI121-FOMT construct was used to inoculate with 2 mL of YEB medium (per liter: 5 g of beef extract, 1 g of yeast extract, 5 g of sucrose, and 0.5 g of MgSO₄·7H₂O), supplemented with 50 mg mL⁻¹ kanamycin and 25 mg mL⁻¹ Rifampicin. The culture was incubated at 28°C until OD₆₀₀ 0.6 -0.8, and the bacteria were pelleted by centrifugation at 5000 rpm for 5 minutes. The cells were washed by MS liquid medium and resuspended pellets with appropriate volume of MS liquid. The tobacco leaf squares were dipped into the bacteria solution for 8 minutes and cultivated on MS media containing 2.0 mg L⁻¹ 6-benzylaminopurine (6-BA), 0.2 mg L⁻¹ 1-naphthylacetic acid (NAA), and 500 mg L⁻¹ ceflomine. The explants were transferred to fresh selected medium after two weeks. When the regenerating plantlets grow to 2 cm, move them to MS media containing 0.2 mg L⁻¹ NAA to induce root. After four weeks, the transgenic tobacco plantlets were transferred to pots and kept in the greenhouse till flowering. The positive transgenic lines were selected by PCR, and transgenic plantlets with empty plasmid were used as control. The anthocyanins in transgenic petals were detected with an HPLC system.

### Functional characterization by transient expression in strawberry fruit

A strawberry cultivar, *Fragaria* × *ananassa* cv. 'Hongyan' (The Beijing Agricultural Technology Extension Station) with fruits turning red were chosen as subjects. A single colony of *Agrobacterium* strain EHA105 with pBI121-FOMT construct was inoculated, and the pellet were centrifuged and washed with infiltration buffer (50 mM Mes, pH 5.6, 2 mM Na₃PO₄, 0.5% glucose (w/v), and 100 mM acetosyringone) according to Hoffmann et al. (Hoffmann, T., G. Kalinowski, and W. Schwab, RNAi-induced silencing of gene expression in strawberry fruit (Fragaria × ananassa) by agroinfiltration: a rapid assay for gene function analysis. Plant Journal, 2006. 48(5): p. 818-826.).

The bacterial suspension was diluted with infiltration buffer to adjust the inoculum concentration to OD₆₀₀ 0.1 - 0.3. A syringe infiltration method was used to transient transform strawberry fruits. The infiltrated fruits were harvested after four days for anthocyanin content analyses as follow.

### Extraction, preparation and analysis of the flavonoids

Appropriate amount of sample (petal, fruits) was powdered with mortars and pestles and extracted for the first time with 2 mL 2% (v/v) formic acid methanol solution shaken in a QL-861 vortex (Kylinbell Lab Instruments, Jiangsu, China), sonicated in KQ-500DE ultrasonic cleaner (Ultrasonic instruments, Jiangsu Kunshan, China) at 20°C for 20 min, centrifuged in SIGMA 3K30 (SIGMA centrifugers, Germany) (12000 rpm, 10 min), and the supernatant was collected. Additional 2 mL and 1 mL extraction solution was supplemented to the residue, and repeated aforesaid operation for the second and third times. All extract was pooled and filtrated through 0.22 µm reinforced nylon membrane filters (Shanghai ANPEL, Shanghai, China) before the HPLC-DAD and HPLC-ESI-MSⁿ analyses. Three replicates were performed for each sample.

The HPLC system was the same with reaction product analysis, the following solvent and gradient were used: A, 10% aqueous formic acid; B, 0.1% formic acid in acetonitrile; constant gradient from 5 to 40% B within 25 min, maintain 40% B for 5 min, and then back to 5% B in 5 min; the flow rate was 0.8 mL min⁻¹; Column temperature was maintained at 35°C; 10 µL of analyte was injected. Chromatograms were obtained at 525 nm for anthocyanins and 350 nm for other flavonoids.

HPLC-ESI-MSⁿ analysis was carried with an Agilent-1100 HPLC system equipped with a UV detector coupled to a LC-MSD Trap VL ion-trap mass spectrometer via an ESI source (Agilent Technologies, Palo Alto, CA). The HPLC separation condition was the same as described above. The MS conditions were listed as follow: negative-ion (NI) mode; capillary voltage of 3.5 kV; a nebulization pressure of 241.3 kPa; and a gas (N₂) temperature of 350°C with flow rate of 6.0 L min⁻¹. Capillary offset voltage was 77.2 V. MS spectra were recorded over the range from *m*/*z* 50 - 1000.

### Results

### Flavonoid O-methyltransferases activity in transgenic tobacco

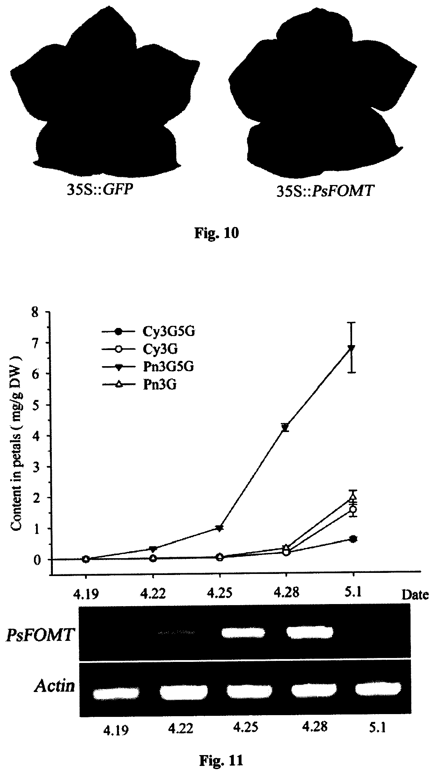

The inventors use transgenic tobacco to characterize the function of *FOMTs in vivo.* The anthocyanins in flower petals of transgenic tobacco lines with the vector 35S::*PsFOMT* and 35S::*PtFOMT* were investigated by the HPLC system. Compared with control harboring the empty vector , in which the main anthocyanin is cyanidin 3-*O*-rutinoside (Cy3R), the 35S::*PsFOMT* and 35S::*PtFOMT* transgenic tobacco petals were detected a new anthocyanin, which has *m*/*z* of 301 and 609, the molecular weight of peonidin and its rutinoside in the positive model (Fig. 9A). The result indicated that *PsFOMT* and *PtFOMT* performed as a methyltransferase in tobacco petal. Moreover, the content of peonidin 3-*O*-rutinoside (Pn3R) in total anthocyanin (TA) was much higher in 35S::*PsFOMT* lines than that of 35S::*PtFOMT* lines (Fig. 9A), suggesting the enzyme of PsFOMT *invivo* was more active than that of PtFOMT, which is agreed with the result of recombinant protein assays. The flower color of 35S::*PsFOMT* lines tend to purplish compared with control lines (Fig. 10), while the *PtFOMT* lines demonstrate no obvious difference on flower color. It is suggesting that methylation could be an approach to flower color variation.

### Flavonoid O-methyltransferases activity in transient strawberry fruit

We used *Agrobacterium*-mediated transient transformation of strawberry fruits to investigate the FOMT activity *in vivo.* This approach allowed us to avoid time-consuming transgenic assay and decipher function of a heterologous gene (Hoffmann, T., G. Kalinowski, and W. Schwab, RNAi-induced silencing of gene expression in strawberry fruit (Fragaria × ananassa) by agroinfiltration: a rapid assay for gene function analysis. Plant Journal, 2006. 48(5): p. 818-826.; Spolaore, S., L. Trainotti, and G. Casadoro, A simple protocol for transient gene expression in ripe fleshy fruit mediated by Agrobacterium. Journal of Experimental Botany, 2001. 52(357): p. 845-850.). According to the former reports and investigation on anthocyanins in strawberry fruit, there are only pelargonidin derivates in *Fragaria* × *ananassa* cv. 'Hongyan', which are not the substrate of PsFOMT for *in vitro* assays. To induce Cy-type anthocyanins accumulation in strawberry fruits, R2R3 MYB transcript factor *PAP1* (*production of anthocyanin pigment 1)* gene (Borevitz, J.O., et al., Activation tagging identifies a conserved MYB regulator of phenylpropanoid biosynthesis. Plant Cell, 2000. 12(12): p. 2383-2393.) was transiently introduced to strawberry fruits along with *FOMTs* by agroinfiltration. Over expression of *PAP1* gene in Arabidopsis, tobacco and tomato has validated the function of anthocyanins accumulation (Borevitz, J.O., et al., Activation tagging identifies a conserved MYB regulator of phenylpropanoid biosynthesis. Plant Cell, 2000. 12(12): p. 2383-2393.; Zhou, L.L., et al., Development of tobacco callus cultures over expressing Arabidopsis PAP1/MYB75 transcription factor and characterization of anthocyanin biosynthesis. Planta, 2008. 229(1): p. 37-51.; Zuluaga, D.L., et al., Arabidopsis thaliana MYB75/PAP1 transcription factor induces anthocyanin production in transgenic tomato plants. Functional Plant Biology, 2008. 35(7): p. 606-618.; Xie, D.Y., et al., Metabolic engineering of proanthocyanidins through co-expression of anthocyanidin reductase and the PAP1 MYB transcription factor. Plant Journal, 2006. 45(6): p. 895-907.). As expected, the *PAP1* transient expression fruit accumulated Cy 3-*O*-glucoside and Cy 3-*O*-malonylglucoside in addition to Pg anthocyanins (Fig. 9B). The anthocyanins in strawberry fruit were listed in Table 5, and each of them was identified by mass spectrometry and literature (da Silva, F.L., et al., Anthocyanin pigments in strawberry. LWT-Food Science and Technology, 2007. 40(2): p. 374-382.). Coexpression assays of *PAP1* and *FOMTs* in strawberry fruits showed that the content of Cy 3-*O*-glucoside and Cy 3-*O*-malonylglucoside decreased, whilst the corresponding methylated anthocyanins Pn 3-*O*-glucoside and Pn 3-*O*-malonylglucoside increased (Fig. 9B). The new compounds were identified by mass spectrometry and credible reference saved in our lab. It is noticed that the conversion efficiency of *PsFOMT* and *PtFOMT* was different. The proportion of methylated products (Pn type) to precursors (Cy type) in *PsFOMT* transient expression fruit is more than that of *PtFOMT*, which is consistent with the recombinant protein results *in vitro.*

**Table 5 The anthocyanin compounds in strawberry fruit**

| | | | |
|---|---|---|---|
| a1 | Cy 3-*O*-glucoside | a5 | Pn 3-*O*-glucoside |
| a2 | Pg 3,5-di-*O*-glucoside | a6 | Cy 3-*O*-malonylglucoside |
| a3 | Pg 3-*O*-glucoside | a7 | Pg 3-*O*-malonylglucoside |
| a4 | Pg 3-*O*-rutinoside | a8 | Pn 3-*O*-malonylglucoside |

### Expression of PsFOMT and anthocyanin accumulation at different developmental phases of subjected flowers

Transcription of *PsFOMT* in *Paeonia suffruticosa* cv. 'Gunpohden' petals at different developmental stages was analyzed by RT-PCR. The gene started to express at the colorless bud stage, then continued to increase to the maximum when the petals was full coloration and began to blossom. When the flower was fully open, the expression of *PsFOMT* was hardly detected. The investigation of anthocyanin accumulation was conducted at the same stages with gene expression analysis. There are four anthocyanins in the petal, including Cy3G, Cy3G5G, Pn3G, and Pn3G5G. The main anthocyanin is Pn3G5G, and it was dominant which is coincident with the gradually increased *PsFOMT* expression (Fig. 11). It is suggested that *PsFOMT* directly related to the accumulation of Pn3G5G in *Paeonia suffruticosa* cv. 'Gunpohden' flower.

### Site-directed mutagenesis and activity

To validate the key amino acid responsible for the activity, site-directed mutagenesis was carried out using the PCR method and Fast Mutagenesis System (TransGen), and primer sequences used are given in Table 1. Four constructs were built following the template sequence of *PtFOMT*, named as PtFOMT-G13E, PtFOMT-T85A, PtFOMT-R87L, and PtFOMT-T205R. The corresponding recombinant proteins were purified and the catalytic activities were examined. The results showed that mutant of PtFOMT-R87L regain the activity equal to PsFOMT. The other mutants have no significant improved activity compared with PtFOMT. The leucine at 87-position is a vital residue for the methyltransferase activity. The reversed mutation of PsFOMT-L87R, PsFOMT-L87A further confirmed the conclusion by possessing low enzyme efficiency similar to PtFOMT (Table 4).

**Table 4 The comparison of activities among recombinant polypeptides with site mutagenesis using Cy3G5G as substrate**

| Incubation 5 min Polypeptide | Activity (%) | ± SD | Incubation 30 min Polypeptide | Activity (%) | ± SD |
|---|---|---|---|---|---|
| PsFOMT | 100 | 0 | PsFOMT | 100 | 0 |
| PtFOMT-G13E | 0 | 0 | PtFOMT-G13E | 18.5 | 0.4 |
| PtFOMT-T85A | 0 | 0 | PtFOMT-T85A | 14.0 | 0.3 |
| PtFOMT-R87L | 100.7 | 1.1 | PtFOMT-R87L | 104.5 | 0.7 |
| PtFOMT-T205R | 0 | 0 | PtFOMT-T205R | 38.1 | 0.6 |
| PsFOMT-L87R | 0 | 0 | PsFOMT-L87R | 40.0 | 0.6 |
| PsFOMT-L87A | 0 | 0 | PsFOMT-L87A | 48.3 | 0.7 |
| PtFOMT | 0 | 0 | PtFOMT | 4.2 | 0.1 |

| | | | | | |
|---|---|---|---|---|---|
| Activity %, the portion of recombinant protein activity compared with PsFOMT; SD, standard deviation with three repetitions. | | | | | |

## Claims

1. A protein with anthocyanin 3'-*O*-methyltransferase or 3',5'-*O*-methyltransferase activity, which is **characterized in that** the protein has an amino acid sequence shown in SEQ ID NO: 3, *or an* amino acid sequence of SEQ ID NO: 3 with a deletion, substitution or insertion of one amino acid in said amino acid sequence.

2. The protein according to claim 1, which is **characterized in that** the anthocyanin 3'-*O-*methyltransferase or 3',5'-*O*-methyltrasnferase activity means the expression wherein the protein facilitates the methylation of an anthocyanin, thereby contributing to the flower color variation.

3. A gene comprising the nucleotide sequence shown in SEQ ID NO: 1 or a nucleotide sequence encoding the protein of SEQ ID NO: 3, which has anthocyanin 3'-*O*-methyltransferase or 3',5'-O-methyltransferase activity.

4. An expression vector comprising the gene of claim 3.

5. A transformant transformed with the expression vector of claim 4.

6. A method for obtaining the transgenic plant expressing an anthocyanin 3'-*O-*methyltransferase or 3',5'-*O*-methyltransferase, comprising the step of introducing a polynucleotide encoding a protein comprising an amino acid sequence of SEQ ID NO: 3 into a target plant tissue to form a transformed target plant tissue; and culturing the transformed target plant tissue to allow expression of the protein comprising the amino acid sequence of SEQ ID NO: 3.

7. The method according to claim 6, which is **characterized in that** the transgenic plant is transgenic tobacco.

8. A transgenic plant, wherein the gene of claim 3 is artificially introduced into the target plant.

9. A method to manipulate the activity of the protein of claim 1 or 2, which is **characterized in that** the mutation of the single amino acid R87L is carried out.

## Patentansprüche

1. Ein Protein mit Anthocyanin-3'-*O*-methyltransferase- oder -3',5'-*O*-methyltransferase-Aktivität, welches **dadurch gekennzeichnet ist, dass** das Protein eine Aminosäuresequenz aufweist, die in SEQ ID Nr.: 3 gezeigt ist, oder eine Aminosäuresequenz der SEQ ID Nr.: 3 mit einer Deletion, Substitution oder Insertion einer Aminosäure in der Aminosäuresequenz aufweist.

2. Das Protein nach Anspruch 1, welches **dadurch gekennzeichnet ist, dass** die Anthocyanin-3'-*O*-methyltransferase- oder -3',5'-*O*-methyltransferase-Aktivität die Expression bedeutet, wobei das Protein die Methylierung eines Anthocyanins erleichtert, wodurch zur Variation der Farbe von Blumen beigetragen wird.

3. Ein Gen, welches die Nukleotidsequenz umfasst, die in SEQ ID Nr.: 1 gezeigt ist, oder eine Nukleotidsequenz umfasst, die das Protein der SEQ ID Nr.: 3 kodiert, welches Anthocyanin-3'-*O*-methyltransferase- oder -3',5'-*O*-methyltransferase-Aktivität aufweist.

4. Ein Expressionsvektor, der das Gen nach Anspruch 3 umfasst.

5. Ein Transformant, der mit dem Expressionsvektor nach Anspruch 4 transformiert wurde.

6. Ein Verfahren zum Erhalt der transgenen Pflanze, die eine an Anthocyanin-3'-*O-*methyltransferase oder -3',5'-*O*-methyltransferase exprimiert, umfassend dem Schritt des Einfügens eines Polynukleotids, das ein Protein kodiert, welches eine Aminosäuresequenz der SEQ ID Nr.: 3 umfasst, in ein Zielpflanzengewebe, um ein transformiertes Zielpflanzengewebe zu bilden; und Züchten des transformierten Zielpflanzengewebes, um die Expression des Protein zu erlauben, welches die Aminosäuresequenz der SEQ ID Nr.: 3 umfasst.

7. Das Verfahren nach Anspruch 6, welches **dadurch gekennzeichnet ist, dass** die transgene Pflanze transgener Tabak ist.

8. Eine transgene Pflanze, wobei das Gen nach Anspruch 3 künstlich in die Zielpflanze eingeführt wurde.

9. Ein Verfahren zur Beeinflussung der Aktivität des Proteins nach Anspruch 1 oder 2, welches **dadurch gekennzeichnet ist, dass** die Mutation der einzelnen Aminosäure R87L durchgeführt wird.

## Revendications

1. Protéine avec une activité d'anthocyanine 3'-*O*-méthyltransférase ou 3',5'-*O*-méthyltransférase, qui est **caractérisée en ce que** la protéine a une séquence d'acides aminés présentée dans la SEQ ID NO : 3, ou une séquence d'acides aminés de la SEQ ID NO : 3 avec une délétion, une substitution ou une insertion d'un acide aminé dans ladite séquence d'acides aminés.

2. Protéine selon la revendication 1, qui est **caractérisée en ce que** l'activité d'anthocyanine 3'-*O*-méthyltransférase ou 3',5'-*O*-méthyltransférase désigne l'expression dans laquelle la protéine facilite la méthylation d'une anthocyanine, contribuant ainsi à la variation de couleur d'une fleur.

3. Gène comprenant la séquence de nucléotides présentée dans la SEQ ID NO : 1 ou une séquence de nucléotides codant pour la protéine de la SEQ ID NO : 3, qui a une activité d'anthocyanine 3'-*O*-méthyltransférase ou 3',5'-*O*-méthyltransférase.

4. Vecteur d'expression comprenant le gène selon la revendication 3.

5. Transformant transformé avec le vecteur d'expression selon la revendication 4.

6. Procédé pour obtenir la plante transgénique exprimant une anthocyanine 3'-*O*-méthyltransférase ou 3',5'-*O*-méthyltransférase, comprenant l'étape consistant à introduire un polynucléotide codant pour une protéine comprenant une séquence d'acides aminés de la SEQ ID NO : 3 dans un tissu de plante cible afin de former un tissu de plante cible transformé ; et à cultiver le tissu de plante cible transformé pour permettre l'expression de la protéine comprenant la séquence d'acides aminés de la SEQ ID NO : 3.

7. Procédé selon la revendication 6, qui est **caractérisé en ce que** la plante transgénique est du tabac transgénique.

8. Plante transgénique, le gène selon la revendication 3 étant introduit artificiellement dans la plante cible.

9. Procédé pour manipuler l'activité de la protéine selon la revendication 1 ou 2, qui est **caractérisé en ce que** la mutation du seul acide aminé R87L est effectuée.
